Europäisches Patentamt

European Patent Office

Office européen des brevets

(19)

(11) EP 0 864 316 B1

(12) FASCICULE DE BREVET EUROPEEN

(45) Date de publication et mention
de la délivrance du brevet:
06.10.1999 Bulletin 1999/40

(51) Int. Cl.$^6$: A61K 7/42, A61K 7/06

(21) Numéro de dépôt: 98400439.0

(22) Date de dépôt: 23.02.1998

(54) **Compositions cosmétiques photoprotectrices contenant un système filtrant les rayons UV, une dispersion de particules de polymère non-filmogène et une phase grasse**

Kosmetische Sonnenschutzmittel enthaltend einen UV-Filter, eine Dispersion von nicht filmbildenden, polymeren Teilchen und eine Ölphase

Cosmetic sunscreen compositions containing a UV filter, a dispersion of non film-forming polymeric particles and an oil phase

(84) Etats contractants désignés:
AT BE CH DE ES FR GB IT LI PT SE

(30) Priorité: 10.03.1997 FR 9702800

(43) Date de publication de la demande:
16.09.1998 Bulletin 1998/38

(73) Titulaire: L'OREAL
75008 Paris (FR)

(72) Inventeurs:
• Plessix, Hervé
92340 Bourg La Reine (FR)
• Mondet, Jean
93600 Aulnay-Sous-Bois (FR)
• De Rigal, Jean
77410 Claye Souilly (FR)

(74) Mandataire: Miszputen, Laurent
L'OREAL-DPI
6 rue Bertrand Sincholle
92585 Clichy Cédex (FR)

(56) Documents cités:
EP-A- 0 268 938          EP-A- 0 503 922
EP-A- 0 681 830          EP-A- 0 755 670
WO-A-95/09895

EP 0 864 316 B1

Printed by Xerox (UK) Business Services
2.16.7/3.6

## Description

La présente invention concerne de nouvelles compositions cosmétiques aqueuses améliorées à usage topique plus particulièrement destinées à la photoprotection des matières kératiniques humaines telles que la peau contre le rayonnement ultraviolet (compositions dénommées ci-après plus simplement compositions anti-solaires) contenant un système filtrant les radiations UV, une dispersion de particules de polymère non-filmogène d'indice de réfraction particulier et une phase grasse d'indice de réfraction particulier ainsi que leurs diverses applications.

Il est connu que le rayonnement lumineux de longueur d'onde 320-400 nm (UVA) permet le brunissement de l'épiderme humain ; ce rayonnement est toutefois susceptible d'induire une altération de celui-ci, notamment dans le cas d'une peau claire ou sensible aux UV, ou d'une peau continuellement exposée. Les UVA provoquent en particulier une perte d'élasticité de la peau et l'apparition de rides conduisant à un vieillissement prématuré.

Il est également connu que le rayonnement lumineux de longueur d'onde 280-320 nm (UVB), provoque des érythèmes et des brûlures cutanées qui peuvent provoquer, par l'intermédiaire de l'inflammation, un certain vieillissement cutané. Il est donc nécessaire, afin de conserver une qualité de peau correcte après une exposition à un rayonnement UV de la protéger pendant l'exposition.

De nombreux composés destinés à la photoprotection des matières kératiniques et en particulier de la peau ont été proposés à ce jour. On peut citer, par exemple, des composés aromatiques hydrophiles ou lipophiles susceptibles d'absorber dans un domaine de longueur d'onde compris dans la zone 280-315 nm et/ou dans la zone 315-400 nm. On connaît également des compositions cosmétiques antisolaires comprenant des nanoparticules d'oxydes minéraux susceptibles d'absorber et/ou réfléchir les UV.

L'efficacité d'une composition antisolaire pour la peau se traduit généralement en terme de facteur de protection solaire (SPF) qui est défini par le rapport de la quantité d'énergie nécessaire pour provoquer un début d'érythème sur la peau protégée par l'agent filtrant les radiations UV sur la quantité d'énergie nécessaire pour provoquer un début d'érythème sur la peau non-protégée.

Certaines substances actives ayant des propriétés filtrantes des radiations UV utilisées dans des formulations antisolaires présentent un potentiel de toxicité vis-à-vis de la peau ou des autres matières kératiniques humaines. D'une façon générale, on cherche à diminuer les risques de toxicité en réduisant dans les formulations antisolaires la teneur en filtre UV tout en conservant leur niveau de protection contre les effets des radiations UV.

On a cherché à atteindre cet objectif dans la demande EP-A-681830 en associant à des systèmes organiques filtrant les radiations UV, un mélange de polymères constitué d'un copolymère d'éthylène/acétate de vinyle et d'un polymère acrylique sous forme de particules, de manière à augmenter le facteur de protection solaire (SPF).

On a cherché à atteindre cet objectif dans la demande EP-A-669124 en utilisant dans une formulation antisolaire l'association d'un système organique filtrant les radiations UV et d'un latex constitué de particules creuses de polymère réticulé en dispersion.

La demanderesse a découvert de manière surprenante qu'une formulation aqueuse antisolaire à base de filtres UV hydrosolubles contenant l'association d'une dispersion de particules de polymère non-filmogène d'indice de réfraction particulier et d'une phase grasse d'indice de réfraction particulier présentait un facteur de protection solaire (SPF) sensiblement amélioré par rapport aux formulations solaires de l'art antérieur associant des filtres UV à des particules de polymère.

Les compositions cosmétiques ou dermatologiques selon l'invention contiennent au moins une phase grasse, une phase aqueuse, un système filtrant les radiations UV hydrosoluble et des particules de polymère non-filmogène et sont caractérisées par le fait que ladite phase grasse, ayant un indice de réfraction $n_1$ et lesdites particules de polymère présentant un indice de réfraction $n_2$ sont choisies de telle sorte que :

$$| n_2 - n_1 | \leq 0,07.$$

Dans la suite de la présente description, on entend par 《système filtrant les radiations UV》 un agent filtrant les radiations UV constitué soit d'un composé unique filtrant les radiations UV soit un mélange de plusieurs composés filtrant les radiations UV, par exemple mélange comprenant un filtre UVA et un filtre UVB.

Dans la suite de la présente description, les matières kératiniques sont choisies parmi la peau, le cuir chevelu, les cheveux, les cils, les sourcils ou les ongles.

Dans la suite de la présente description, les indices de réfraction $n_1$ et $n_2$ relatifs respectivement aux huiles constituant la phase grasse et aux particules de polymère non-filmogène sont mesurés avec un réfractomètre conventionnel dans lequel la source lumineuse utilisée correspond à la raie du sodium à la température ambiante.

Les compositions de l'invention comprennent au moins un polymère non-filmifiable sous forme de particules en dispersion dans un milieu aqueux et/ou organique. On peut ainsi utiliser en particulier un milieu dispersant hydroalcoolique, alcoolique ou huileux.

Le polymère non-filmogène utilisé doit se présenter sous forme de particules solides en suspension dans le milieu considéré, ce qui exclut l'utilisation de polymères solubles dans ledit milieu.

On peut utiliser tout type de polymère répondant à ces critères. On peut ainsi employer des polymères radicalaires, des polycondensats, des polymères d'origine naturelle éventuellement modifiés. On peut citer, par exemple, les polyesters, les polyesteramides, les alkydes, les polyacryliques, les polyvinyliques, les polyuréthanes, les polystyrènes, les polymères carbohydratés naturels ou modifiés et leurs dérivés, les protéines naturelles ou naturelles modifiées telles que les protéines globulaires naturelles ou naturelles modifiées, seuls ou en mélange.

De façon préférentielle, on utilise des polymères réticulés par exemple à l'aide d'agents réticulants multifonctionnels. Ces polymères présentent l'avantage de rester sous forme de particules non filmifiables et d'être insolubles dans le milieu considéré, quelle que soit la nature chimique du polymère de base. On peut citer par exemple les polyacryliques réticulés par des acrylates ou méthacrylates d'éthylèneglycol, de diéthylèneglycol ou d'alkylènes ou encore des polycondensats réticulés par des polyisocyanates.

La dispersion contient de préférence de 30 à 60% en poids de matière sèche de polymère.

Les particules de polymère non-filmogène dispersées ont, d'une manière avantageuse, une taille allant de 3 à 700 nm et plus préférentiellement de 10 à 350 nm, ce qui permet d'une part d'éviter la solubilisation des particules dans le milieu et d'autre part d'améliorer la stabilité de la composition dans laquelle les particules ne décantent pas tout en diffusant correctement le rayonnement.

Dans les compositions selon l'invention, le polymère est de préférence présent à raison de 0,5 à 30% en poids en matière sèche et plus particulièrement de 2 à 15% en poids par rapport au poids total de la composition.

La dispersion de particules de polymère non-filmogène peut être préparée selon les techniques connues dans l'art antérieur. En particulier, on peut citer la polymérisation en émulsion permettant d'obtenir des particules élémentaires de taille variable avec une très faible polydispersité autour de cette taille.

Les compositions conformes à l'invention contiennent des systèmes filtrant les radiations UV hydrosolubles parmi lesquels on peut citer, par exemple, l'acide para-amino-benzoïque et ses sels, l'acide anthranilique et ses sels, l'acide salicylique et ses sels, les dérivés de l'acide cinnamique et ses sels, les dérivés sulfoniques de benz-x-azole (benzothioazoles, benzimidazoles, benzoxazoles) et leurs sels, les dérivés sulfoniques du benzophénone et leurs sels, les dérivés sulfoniques de benzylidène camphre et leurs sels, les dérivés de benzylidène camphre substitués par une amine quaternaire et leurs sels, les dérivés des acides phtalydène-camphosulfoniques et leurs sels, les dérivés sulfoniques de benzotriazole, les nanoparticules d'oxydes minéraux traitées en surface pour être hydrophiles, leurs mélanges.

On peut également utiliser des polymères hydrophiles présentant, en outre et de par leur nature chimique, des propriétés de photoprotection contre le rayonnement UV. On peut citer les polymères comportant des groupements benzylidène camphre et/ou benzotriazole, substitués ou non. Il est toutefois à noter que le SPF de ces polymères n'est pas suffisant pour que l'on puisse les considérer comme des filtres UV.

Le ou les filtres UV hydrophiles sont présents dans les compositions de l'invention à des concentrations variant de préférence de 0,1 à 30% en poids, plus particulièrement de 0,5 à 25% en poids, par rapport au poids total de la composition.

La phase grasse des compositions selon l'invention peut comporter une ou plusieurs huiles choisies de préférence dans le groupe constitué par :

- les huiles minérales telles que l'huile de paraffine et de vaseline,
- les huiles hydrocarbonées comportant des noyaux aromatiques tels que les esters d'acide benzoïque comme les produits commerciaux FINSOLV vendus par la société

  FINETEX comme ceux définis ci-dessus :
  FINSOLV TN : $C_{12}$-$C_{15}$ alkylbenzoate
  FINSOLV SB : isostéarylbenzoate
  FINSOLV BOD : octyl dodecyl benzoate

- les esters polyglycérolés tels que le polyglycéryl-2-diisostéarate, le polyglycéryl-2-triisostéarate ;
- les huiles d'origine animale telles que le perhydrosqualène, le squalane ;
- les huiles d'origine végétale telles que l'huile d'amande douce, l'huile d'avocat, l'huile de ricin, l'huile d'olive, l'huile de jojoba, l'huile de sésame, l'huile d'arachide, l'huile de macadamia, l'huile de pépins de raisin, l'huile de colza, l'huile de coprah, l'huile de rosiermuscat , l'huile de trioléïne et plus particulièrement les triglycérides dont la teneur en acides gras insaturés est importante ;
- les huiles synthétiques telles que l'huile de purcellin, les isoparaffines ;
- les huiles fluorées et perfluorées telles que la perfluorodécaline, les polyéthers perfluorés comme les produits FOMBLIN HC 04, FOMBLIN HC 25, FOMBLIN HC R vendus par la société MONTEDISON ;
- les esters d'acides gras tels que l'huile de Purcellin, l'octyldodécyl néodécanoate; l'isodécyl isononanoate, l'isononyl isononanoate, l'octyl dodécyl néopentanoate, le myristate d'isopropyle, le palmitate d'isopropyle ;
- les polyoléfines de poids moléculaires élevés tels que les polybutènes ou les polydécènes.
- les alcools gras tels que l'octyldodécanol et l'alcool oléïque ;
- les silicones volatiles ou non-volatiles, linéaires, ramifiées ou cycliques, organomodifiées ou non telles que le produit SILBIONE 70 641 V200 (diphényldiméthicone) vendu par RHONE POULENC ou les produits DC 556 (phényltriméthicone) et SF 558 (méthyl phényl polysiloxane) vendu par DOW CORNING ; les silicones cycliques volatiles ou les polydiméthylsiloxanes linéaires non-volatiles.

De façon connue, toutes les compositions de l'invention peuvent contenir des adjuvants habituels dans les domaines cosmétique et dermatologique, d'autres gélifiants et/ou épaississants classiques ; des polymères ; des agents hydratants ; des émollients ; des actifs hydrophiles ou lipophiles comme des céramides ; des agents anti-radicaux libres ; des agents amincissants ; des bactéricides ; des séquestrants ; des antipelliculaires ; des antioxydants ; des conservateurs ; des agents alcanisants ou acidifiants ; des parfums ; des charges ; des cires minérales, synthétiques, végétales ou animales, des corps gras pâteux ; des solvants organiques ; des adoucissants ; des filtres solaires complémentaires ; des agents anti-mousses ; des conservateurs, des tensioactifs, des charges, des auto-bronzants, des propulseurs ; des colorants, des pigments. Les quantités de ces différents adjuvants sont celles classiquement utilisées dans les domaines considérés.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention peuvent se présenter sous forme de sérum, de lait, de crèmes plus ou moins onctueuses, de pâte. Ces compositions sont préparées selon les méthodes usuelles.

Les compositions selon l'invention peuvent se présenter sous forme liquide, pâteuse ou solide, par exemple sous forme de gel, d'une émulsion huile-dans-eau ou eau-dans-huile, de pommade ou sous forme de mousse.

Les compositions selon l'invention peuvent être utilisées comme produits pour le soin telles que des crèmes ; comme produits de maquillage tels que des fonds de teints, des fards, des rouges à lèvres, des mascaras, des eyeliners ; comme produits capillaires tels que des shampooings, des lotions ou des gels rincés ou non-rincés.

Les compositions selon l'invention peuvent être utilisées comme produits pour la protection des matières kératiniques contre les rayonnement UV et en particulier de la peau.

L'invention a également pour objet un procédé cosmétique de protection des matières kératiniques humaines en particulier de la peau contre les rayonnements UV, consistant à appliquer une quantité efficace d'une composition telle que définie ci-dessus sur le support kératinique.

Les exemples qui suivent servent à illustrer l'invention sans toutefois présenter un caractère limitatif.

**EXEMPLE DE PREPARATION A** :

**Préparation d'une dispersion de particules de polymère acrylique non-filmifiables**

Composition du polymère:

| - méthacrylate de méthyle | 91 % en poids |
|---|---|
| - acide méthacrylique | 5 % en poids |
| - diméthacrylate d'éthylène glycol (réticulant) | 4 % en poids |

**Mode opératoire** :

Dans un réacteur équipé d'une agitation mécanique centrale, d'un thermomètre et d'un réfrigérant, on introduit 100 g d'eau permutée, 16 g en matière active d'un tensioactif alkyl éthoxy sulfate vendu sous le nom d'ABEX JKB par la Société RHONE POULENC et 2,5 g de persulfate de potassium. On porte le mélange sous agitation rapide à une température de 80°C.

On prépare en parallèle les deux solutions dites 《《coulées》》 $S_1$ et $S_2$ suivantes :

coulée S$_1$ (solution de monomères) :

| - Méthacrylate de méthyle | 1820 g |
|---|---|
| - Acide méthacrylique | 100 g |
| - Diméthacrylate d'éthylène glycol (réticulant) | 80g |

coulée S$_2$ :

| - Eau permutée | 5000 g |
|---|---|
| - ABEX JKB | 160 g |
| - Persulfate de potassium | 7,5 g |

Lorsque la solution aqueuse dans le réacteur est parvenue à 80°C, on ajoute 10% de la solution S$_1$ et on laisse réagir 15 minutes. On coule alors pendant une durée de 4 heures, avec un débit constant et simultanément le reste de la solution S$_1$ et la solution S$_2$. A la fin des deux ajouts simultanés, on élève la température du milieu réactionnel à 85°C et on maintient à cette température pendant 30 minutes.

On laisse refroidir à température ambiante sous agitation. On filtre sur toile de nylon.

On obtient une dispersion de particules de polymère présentant les caractéristiques suivantes :

- Taille moyenne des particules : 110 nm
- Polydispersité en taille de particules mesurée par diffusion quasi-élastique de la lumière avec un appareil du type COULTER N4 SD : < 0,1.
- Extrait sec en étuve ventilée à 80°C jusqu'à poids constant : 27%.
- indice de réfraction n$_2$ = 1,49.

**EXEMPLE DE PREPARATION B :**

**Préparation d'une dispersion de particules de polystyrène réticulé non-filmifiables**

Dans un réacteur cylindrique de 1 litre avec agitation centrale, réfrigérant, thermomètre et barbotage d'azote, on introduit les réactifs suivants :

| - Styrène | 98 g |
|---|---|
| - Divinylbenzène (réticulant) | 2 g |
| - Nonylphénol polyoxyéthyléné à 25 OE vendu sous le nom AD 33 par SEPPIC | 0,0735 g M.A. |
| - Persulfate de potassium | 0,175 g |
| - Eau permutée | 70 g |

On réalise d'abord la solution aqueuse avec l'eau, le tensioactif et le réticulant sous agitation et barbotage d'azote puis on ajoute le monomère. On règle l'agitation à 300 tours/min. On chauffe le milieu réactionnel à 72°C et on maintient à cette température pendant 6 heures. On élève ensuite la température à 85°C pendant 1 heure. On ramène à température et on filtre sur tamis.

On obtient une dispersion de particules de polymère présentant les caractéristiques suivantes :

- Taille moyenne des particules : 60 nm
- Polydispersité en taille de particules mesurée par diffusion quasi-élastique de la lumière avec un appareil du type COULTER N4 SD : < 0,1.
- Extrait sec en étuve ventilée à 80°C jusqu'à poids constant : 27%.
- indice de réfraction $n_2$ = 1,59.

## EXEMPLES COMPARATIFS DE FORMULATIONS SOLAIRES

On prépare les cinq émulsions huile-dans-eau suivantes 1 à 5 :

### EMULSION SOLAIRE 1 (Invention)

| | |
|---|---|
| - Latex polyacrylique selon l'exemple A ($n_2$ = 1,49) | 10% en poids (*M.A.) |
| - Huile de ricin ($n_1$ = 1,48) | 20 % en poids |
| - Acide 4-(4,7,7-triméthyl-3-oxo-bicyclo-[2.2.1]-hept-2-ylidèneméthyl) benzène sulfonique vendu sous le nom MEXORYL SL par CHIMEX en solution aqueuse à 50 % dont le pH est ajusté à 7 par la triéthanolamine (Filtre UV hydrosoluble) | 10% en poids |
| - Tensioactif polyoxyéthyléné et polyoxypropyléné vendu sous le nom SYMPERONIC PE/P85 par ICI AMERICAS | 5 % en poids |
| - Eau | 55 % en poids |

(*) M.A. = matière active

### EMULSION SOLAIRE 2 (Hors invention)

| | |
|---|---|
| - Latex polystyrène selon l'exemple B ($n_2$ = 1,59) | 10% en poids (M.A.) |
| - Huile de ricin ($n_1$ = 1,48) | 20 % en poids |
| - Acide 4-(4,7,7-triméthyl-3-oxo-bicyclo-[2.2.1]-hept-2-ylidèneméthyl) benzène sulfonique vendu sous le nom MEXORYL SL par CHIMEX en solution aqueuse à 50% dont le pH est ajusté à 7 par la triéthanolamine (Filtre UV hydrosoluble) | 10% en poids |
| - Tensioactif polyoxyéthyléné et polyoxypropyléné vendu sous le nom SYMPERONIC PE/P85 par ICI AMERICAS | 5 % en poids |
| - Eau | 55 % en poids |

### EMULSION SOLAIRE 3 (Hors invention)

| | |
|---|---|
| - Latex polystyrène selon l'exemple B ($n_2$ = 1,59) | 10% en poids (M.A.) |

(suite)

| | |
|---|---|
| - Adipate de diisopropyle ($n_1$ = 1,423) | 20 % en poids |
| - Acide 4-(4,7,7-triméthyl-3-oxo-bicyclo-[2.2.1]-hept-2-ylidèneméthyl) benzène sulfonique vendu sous le nom MEXORYL SL par CHIMEX en solution aqueuse à 50% dont le pH est ajusté à 7 par la triéthanolamine (Filtre UV hydrosoluble) | 10% en poids |
| - Tensio-actif polyoxyéthyléné et polyoxypropyléné vendu sous le nom SYMPERONIC PE/P85 par ICI AMERICAS | 5 % en poids |
| - Eau | 55 % en poids |

**EMULSION SOLAIRE 4 sans latex (Hors invention)**

| | |
|---|---|
| - Huile de ricin ($n_1$ = 1,48) | 20 % en poids |
| - Acide 4-(4,7,7-triméthyl-3-oxo-bicyclo-[2.2.1]-hept-2-ylidène-méthyl) benzène sulfonique vendu sous le nom MEXORYL SL par CHIMEX en solution aqueuse à 50% dont le pH est ajusté à 7 par la triéthanolamine (Filtre UV hydrosoluble) | 10% en poids |
| - Tensio-actif polyoxyéthyléné et polyoxypropyléné vendu sous le nom SYMPERONIC PE/P85 par ICI AMERICAS | 5 % en poids |
| - Eau | 65 % en poids |

**EMULSION 5 sans filtre UV (Hors invention)**

| | |
|---|---|
| - Latex polyacrylique selon l'exemple A ($n_2$ = 1,49) | 10% en poids (M.A.) |
| - Adipate de diisopropyle ($n_1$ = 1,423) | 20 % en poids |
| - Tensioactif polyoxyéthyléné et polyoxypropyléné vendu sous le nom SYMPERONIC PE/P85 par ICI AMERICAS | 5 % en poids |
| - Eau | 65 % en poids |

**Mode de préparation des émulsions 1 à 3 et 5**

Dans un flacon à vis de 30 ml, le tensioactif est dispersé à température ambiante dans l'eau en présence du filtre UV hydrosoluble. L'huile est chauffée à 70°C puis versée dans la phase aqueuse. On agite à 70°C les constituants présents dans le mélange obtenu au moyen d'un dispositif d'agitation mécanique pendant 5 minutes à 18000 tours/minute. On laisse ensuite revenir l'émulsion à température ambiante. Celle-ci est ensuite traitée aux ultra-sons. On alterne pulsion et arrêt chaque seconde pendant 10 minutes. On ajoute le latex, à température ambiante, à la fin de la préparation de l'émulsion, puis on homogénéise le tout avec un appareil d'homogénéisation du type VORTEX.

**TESTS SUR LE FACTEUR DE PROTECTION SOLAIRE**

**a) Principe général :**

On détermine le facteur de protection de la peau vis à vis des rayonnements UV relatif à chacune des émulsions 1 à 5 telles que définies ci-dessus selon la méthode et l'appareillage décrits dans la demande de brevet français publiée FR-A-2 719 989.
Selon cette méthode, l'enregistrement du spectre s'effectue in vivo par réflexion. Quelle que soit la lumière incidente sur la peau, une partie est réfléchie spéculairement par la surface dans toutes les directions de l'espace et ne contient aucune information spectrale ; une autre partie pénètre dans la peau, est diffusée sur les structures internes et en ressort de façon isotrope. Cette énergie contient l'information spectrale des couches traversées et en particulier de la formulation filtrante appliquée à la surface. C'est cette énergie qu'il faut sélectionner et mesurer. On utilise la lumière polarisée.

La lumière incidente E($\lambda$) est polarisée dans le plan d'incidence, la lumière diffusée ED($\lambda$) est mesurée perpendiculairement à la surface de la peau à la sortie d'un polariseur perpendiculaire au plan d'incidence et la lumière réfléchie ER($\lambda$) est mesurée à la sortie d'un polariseur parallèle par rapport au plan d'incidence. On détermine alors un spectre d'absorbance de la peau appelé 〈〈spectre de pseudo-brillance〉〉 par le rapport ER($\lambda$)/ED($\lambda$).

L'appareillage est constitué d'une lampe XENON suivie d'un monochromateur qui est couplé à une fibre optique rentrant dans la sonde de mesure. De cette sonde, sortent deux fibres optiques conduisant respectivement la lumière diffusée et la lumière réfléchie vers des photomultiplicateurs et leur électronique associée. L'appareillage est piloté par un ordinateur.

On calcule le facteur de protection de chaque composition solaire selon la formule suivante :

$$I = \Sigma_i \; \alpha_i \; DB_i \; K_i$$

où $\alpha_i$ désigne le coefficient d'éfficacité érythémale selon la norme de COLIPA dans l'intervalle de longueurs d'onde i donné ; DBi est la variation de pseudo-brillance dans l'intervalle de longueurs d'onde i donné et Ki est le facteur correctif permettant de convertir le spectre de la lampe utilisée en spectre solaire dans l'intervalle de longueur d'onde i donné.

Les coefficients $\alpha_i$ et Ki sont bien connus de la littérature relative aux filtres solaires et sont décrits notamment dans l'article 〈〈A comparison of vivo and in vitro testing of sunscreen formula〉〉 dans Photochem et Photobiol, 1978, vol 29 pp 559-566 et dans le fascicule 〈〈SPF test methods〉〉 janvier 1984 du COLIPA.

Le facteur de protection I ainsi obtenu peut être comparé à l'indice de protection (SPF : Sun Protection Factor) qui est couramment affecté aux produits solaires commercialisés.

#### b) Protocole expérimental :

Les mesures sont effectuées sur l'avant-bras face interne d'un groupe de personnes. Deux zones de mesure sont définies sur chaque bras et délimitées de manière précise à l'aide des électrodes nécessaires au maintien et au repositionnement de la sonde.

Chacune des émulsions 1 à 5 telles que décrites plus haut est appliquée sur chaque zone à raison de 2 mg/cm$^2$ à l'aide d'une micropipette GILSON (48 $\mu$l pour 48 cm$^2$). Avant chaque application, le produit à tester est réhomogénéisé par agitation mécanique.

L'intervalle i de longueurs d'onde utilisé est 280-400 nm. Les mesures de spectre de brillance sont effectuées dans ledit domaine par pas de 5 nm. Elles sont réalisées avant application et 15 minutes après application afin de diminuer les fluctuations des résultats obtenus. Les séances de mesures sont espacées d'au moins 48 heures afin de retrouver une peau sans aucune trace résiduelle de produit.

Tous les produits solaires ont été testés sur le même groupe d'individus afin que les résultats obtenus ne soient pas affectés par d'éventuels effets individus.

On calcule pour chaque émulsion 1 à 5, le facteur I de protection solaire moyen sur le groupe de personnes testées. Les résultats sont indiqués dans le tableau suivant :

| Emulsion testée | Indice de réfraction n$_1$ du latex utilisé | Indice de réfraction n$_2$ de la phase grasse | \| n$_2$ - n$_1$ \| | Facteur de protection solaire moyen $\Sigma_i \; \alpha_i$ DB$_i$ K$_i$ |
|---|---|---|---|---|
| 1 (invention) | 1,49 | 1,48 | 0,01 | 78 |
| 2 (hors invention) | 1,59 | 1,48 | 0,11 | 22 |
| 3 (hors invention) | 1,59 | 1,42 | 0,17 | 27 |
| 4 sans latex (hors invention) | - | 1,48 | - | 23 |
| 5 sans filtre UV (hors invention) | 1,49 | 1,42 | 0,01 | 2,6 |

Ces résultats montrent que l'émulsion solaire 1 selon l'invention contenant un latex d'indice de réfraction $n_1$ et une phase grasse d'indice de réfraction $n_2$ choisis de telle sorte que $|n_2 - n_1| \leq 0{,}07$ (ie : 0,01) présente un facteur de protection solaire moyen considérablement plus élevé que celui des émulsions solaires 2 et 3 contenant un latex et une phase grasse tels que $|n_2 - n_1|$ est respectivement égal à 0,11 et 0,17.

Ces résultats montrent également que les émulsions solaires 2 et 3 contenant un latex et une phase grasse tels que $|n_2 - n_1|$ est égal à respectivement 0,11 et 0,17 présentent des facteurs de protection solaire moyens qui sont équivalents à celui de l'émulsion solaire 4 ne comportant pas de latex. Autrement dit, les associations latex /phase grasse utilisées dans les émulsions 2 et 3 ne permettent pas d'augmenter le facteur de protection solaire d'une émulsion à base d'un filtre UV hydrosoluble.

Ces résultats montrent enfin que l'association latex /phase grasse conforme à l'invention utilisée dans une émulsion ne contenant pas de filtre UV hydrosoluble n'a pratiquement aucun effet d'absorption des radiations UV.

**Revendications**

1.  Composition cosmétique ou dermatologique contenant au moins une phase grasse, une phase aqueuse, un système filtrant les radiations UV hydrosoluble et des particules de polymère non-filmogène, caractérisée par le fait que ladite phase grasse ayant un indice de réfraction $n_1$ et lesdites particules de polymère non-filmogène ayant un indice de réfraction $n_2$ sont choisies de telle sorte que :

$$|n_2 - n_1| \leq 0{,}07.$$

2.  Composition selon la revendication 1, caractérisée par le fait que le polymère non-filmogène est sous forme de particules en dispersion dans un milieu aqueux et/ou organique.

3.  Composition selon la revendication 1 ou 2, caractérisée par le fait que le polymère non-filmogène est choisi parmi les polymères radicalaires, les polycondensats, les polymères d'origine naturelle éventuellement modifiés choisis de telle sorte que $|n_2 - n_1| \leq 0{,}07$.

4.  Composition selon la revendication 3, , caractérisée par le fait que le polymère non-filmogène est choisi parmi les polyesters, les polyesteramides, les alkydes, les polyacryliques, les polyvinyliques, les polyuréthanes, les polystyrènes, les polymères carbohydratés naturels ou modifiés et leurs dérivés, les protéines naturelles ou naturelles modifiées telles que les protéines globulaires naturelles ou naturelles modifiées, seuls ou en mélange.

5.  Composition selon l'une quelconque des revendications 1 à 4, caractérisée par le fait que le polymère non-filmogène est réticulé.

6.  Composition selon l'une quelconque des revendications 1 à 5, caractérisée par le fait que le polymère non-filmogène est sous forme de dispersion aqueuse, hydroalcoolique, alcoolique ou de dispersion dans une phase grasse.

7.  Composition selon l'une quelconque des revendications 1 à 6, caractérisée par le fait que les particules de polymère non-filmogène dispersées ont une taille allant de 3 à 700 nm et plus préférentiellement de 10 à 350 nm.

8.  Composition selon l'une quelconque des revendications 1 à 7, caractérisée par le fait que le polymère non-filmogène est présent à raison de 0,5 à 30% en poids en matière sèche et plus particulièrement de 2 à 15% en poids par rapport au poids total de la composition.

9.  Composition selon l'une quelconque des revendications 1 à 8, caractérisée par le fait que les systèmes filtres UV hydrosolubles sont choisis dans le groupe constitué par l'acide para-aminobenzoïque et ses sels, l'acide anthranilique et ses sels, l'acide salicylique et ses sels, les dérivés de l'acide cinnamique et ses sels, les dérivés sulfoniques de benzothiazole et leurs sels, les dérivés sulfoniques de benzimidazole et leurs sels, les dérivés sulfoniques de benzoxazole et leurs sels, les dérivés sulfoniques du benzophénone et leurs sels, les dérivés sulfoniques de benzylidène camphre et leurs sels, les dérivés de benzylidène camphre substitués par une amine quaternaire et leurs sels, les dérivés des acides phtalydène-camphosulfoniques et leurs sels, les dérivés sulfoniques de benzotriazole, les nanoparticules d'oxydes minéraux traitées en surface pour être hydrosolubles, les polymères hydrosolubles présentant, en outre et de par leur nature chimique, des propriétés de photoprotection contre le rayonnement UV, et leurs mélanges.

10. Composition selon l'une quelconque des revendications 1 à 10, caractérisée par le fait que le système filtre UV hydrosoluble est présent à des concentrations variant de préférence de 0,1 à 30% en poids, plus particulièrement de 0,5 à 25% en poids, par rapport au poids total de la composition.

**11.** Composition selon l'une quelconque des revendications 1 à 11, caractérisée par le fait que la phase grasse comporte une ou plusieurs huiles choisies dans le groupe constitué par :

- les huiles minérales :
- les huiles hydrocarbonées comportant des noyaux aromatiques
- les esters polyglycérolés ;
- les huiles d'origine animale ;
- les huiles d'origine végétale ;
- les huiles synthétiques ;
- les huiles fluorées et perfluorées ;
- les esters d'acides gras ;
- les alcools gras ;
- les silicones volatiles ou non-volatiles, linéaires, ramifiées ou cycliques, organomodifiées ou non.

**12.** Composition selon l'une quelconque des revendications 1 à 12, caractérisée par le fait qu'elle est utilisée comme produit pour le soin ; comme produit de maquillage; comme produit capillaire rincé ou non-rincé.

**13.** Composition selon l'une quelconque des revendications 1 à 12, caractérisée par le fait qu'elle est utilisée comme produit pour la protection des matières kératiniques contre les rayonnement UV et en particulier de la peau.

**14.** Procédé de protection cosmétique des matières kératiniques humaines en particulier de la peau contre les rayonnements UV, consistant à appliquer une quantité efficace d'une composition selon l'une quelconque des revendications 1 à 14 sur le support kératinique.

**Claims**

**1.** Cosmetic or dermatological composition containing at least one fatty phase, an aqueous phase, a water-soluble system for screening out UV radiation and particles of non-film-forming polymer, characterized in that the said fatty phase, which has a refractive index $n_1$, and the said particles of non-film-forming polymer, which have a refractive index $n_2$, are chosen such that:

$$n_2 - n_1 \leq 0.07.$$

**2.** Composition according to Claim 1, characterized in that the non-film-forming polymer is in the form of particles dispersed in an aqueous and/or organic medium.

**3.** Composition according to Claim 1 or 2, characterized in that the non-film-forming polymer is chosen from radical polymers, polycondensates and optionally modified polymers of natural origin chosen such that $n_2 - n_1 \leq 0.07$.

**4.** Composition according to Claim 3, characterized in that the non-film-forming polymer is chosen from polyesters, polyesteramides, alkyds, polyacrylics, polyvinylics, polyurethanes, polystyrenes, natural or modified carbohydrate polymers and derivatives thereof, natural or modified natural proteins such as natural or modified natural globular proteins, alone or as a mixture.

**5.** Composition according to any one of Claims 1 to 4, characterized in that the non-film-forming polymer is crosslinked.

**6.** Composition according to any one of Claims 1 to 5, characterized in that the non-film-forming polymer is in the form of an aqueous, aqueous-alcoholic or alcoholic dispersion or in the form of a dispersion in a fatty phase.

**7.** Composition according to any one of Claims 1 to 6, characterized in that the dispersed particles of non-film-forming polymer have a size ranging from 3 to 700 nm and more preferably from 10 to 350 nm.

**8.** Composition according to any one of Claims 1 to 7, characterized in that the non-film-forming polymer is present in a proportion of from 0.5 to 30% by weight of solids and more particularly from 2 to 15% by weight relative to the total weight of the composition.

9. Composition according to any one of Claims 1 to 8, characterized in that the water-soluble UV screening systems are chosen from the group consisting of para-aminobenzoic acid and salts thereof, anthranilic acid and salts thereof, salicylic acid and salts thereof, cinnamic acid derivatives and salts thereof, sulphonic derivatives of benzothiazole and salts thereof, sulphonic derivatives of benzimidazole and salts thereof, sulphonic derivatives of benzoxazole and salts thereof, sulphonic derivatives of benzophenone and salts thereof, sulphonic derivatives of benzylidenecamphor and salts thereof, benzylidene-camphor derivatives substituted by a quaternary amine and salts thereof, phthalydene-camphorsulphonic acid derivatives and salts thereof, sulphonic derivatives of benzotriazole, nanoparticles of inorganic oxides surface-treated so as to be water-soluble, water-soluble polymers having, in addition and on account of their chemical nature, properties of photoprotection against UV radiation, and mixtures thereof.

10. Composition according to any one of Claims 1 to 10, characterized in that the water-soluble UV screening system is present at concentrations preferably ranging from 0.1 to 30% by weight, more particularly from 0.5 to 25% by weight, relative to the total weight of the composition.

11. Composition according to any one of Claims 1 to 11, characterized in that the fatty phase contains one or more oils chosen from the group consisting of:

- mineral oils;
- hydrocarbon oils containing aromatic rings;
- polyglycerol esters;
- oils of animal origin;
- oils of plant origin;
- synthetic oils;
- fluoro and perfluoro oils;
- fatty acid esters;
- fatty alcohols;
- linear, branched or cyclic, volatile or non-volatile silicones which may or may not be organomodified.

12. Composition according to any one of Claims 1 to 12, characterized in that it is used as a care product; as a make-up product; as a rinse-out or leave-in hair product.

13. Composition according to any one of Claims 1 to 12, characterized in that it is used as a product for protecting keratin substances against UV radiation, and in particular the skin.

14. Process for the cosmetic protection of human keratin substances, in particular the skin, against UV radiation, this process consisting in applying an effective amount of a composition according to any one of Claims 1 to 14 to the keratin support.

**Patentansprüche**

1. Kosmetische oder dermatologische Zusammensetzung, die mindestens eine Fettphase, eine wässerige Phase, ein wasserlösliches System, das UV-Strahlung filtert, und Partikel eines nicht filmbildenden Polymers enthält, dadurch gekennzeichnet, daß die Fettphase mit einem Brechungsindex $n_1$ und die Partikel des nicht filmbildenden Polymers mit einem Brechungsindex $n_2$ so ausgewählt sind, daß gilt:

$$| n_2 - n_1 | \leq 0{,}07.$$

2. Zusammensetzung nach Anspruch 1, dadurch gekennzeichnet, daß das nicht filmbildende Polymer in Form von Partikeln vorliegt, die in einem wässerigen und/oder organischen Medium dispergiert sind.

3. Zusammensetzung nach Anspruch 1 oder 2, dadurch gekennzeichnet, daß das nicht filmbildende Polymer ausgewählt ist unter durch radikalische Polymerisation hergestellten Polymeren, Polykondensaten, Polymeren natürlichen Ursprungs, die gegebenenfalls modifiziert sind und die so ausgewählt sind, daß $|n_2 - n_1| \leq 0{,}07$.

4. Zusammensetzung nach Anspruch 3, dadurch gekennzeichnet, daß das nicht filmbildende Polymer unter Polyestern, Polyesteramiden, Alkydharzen, Polyacrylverbindungen, Polyvinylverbindungen, Polyurethanen, Polystyrolen, natürlichen oder modifizierten kohlenwasserstoffhaltigen Polymeren und ihren Derivaten und natürlichen oder modifizierten natürlichen Proteinen, wie natürlichen oder modifizierten natürlichen globulären Proteinen, ausgewählt ist, die allein oder im Gemisch verwendet werden.

5.  Zusammensetzung nach einem der Ansprüche 1 bis 4, dadurch gekennzeichnet, daß das nicht filmbildende Polymer vernetzt ist.

6.  Zusammensetzung nach einem der Ansprüche 1 bis 5, dadurch gekennzeichnet, daß das nicht filmbildende Polymer in Form einer wässerigen, wässerig-alkoholischen oder alkoholischen Dispersion oder dispergiert in einer Fettphase vorliegt.

7.  Zusammensetzung nach einem der Ansprüche 1 bis 6, dadurch gekennzeichnet, daß die dispergierten Partikel des nicht filmbildenden Polymers eine Größe im Bereich von 3 bis 700 nm und noch bevorzugter von 10 bis 350 nm aufweisen.

8.  Zusammensetzung nach einem der Ansprüche 1 bis 7, dadurch gekennzeichnet, daß das nicht filmbildende Polymer in einem Mengenanteil von 0,5 bis 30 Gew.-% Trockensubstanz und insbesondere von 2 bis 15 Gew.-%, bezogen auf das Gesamtgewicht der Zusammensetzung, vorliegt.

9.  Zusammensetzung nach einem der Ansprüche 1 bis 8, dadurch gekennzeichnet, daß die wasserlöslichen UV-Filtersysteme ausgewählt sind unter p-Aminobenzoesäure und ihren Salzen, Anthranilsäure und ihren Salzen, Salicylsäure und ihren Salzen, Zimtsäurederivaten und ihren Salzen, Sulfonsäurederivaten von Benzothiazol und ihren Salzen, Sulfonsäurederivaten von Benzimidazol und ihren Salzen, Sulfonsäurederivaten von Benzoxazol und ihren Salzen, Sulfonsäurederivaten von Benzophenon und ihren Salzen, Sulfonsäurederivaten von Benzylidencampher und ihren Salzen, Benzylidencampher-Derivaten, die mit einem quartären Amin substituiert sind, und ihren Salzen, Derivaten von Phthalydencamphersulfonsäuren und ihren Salzen, Sulfonsäurederivaten von Benzotriazol, anorganischen Nanopartikeln von Oxiden, die durch Oberflächenbehandlung wasserlöslich gemacht sind, wasserlöslichen Polymeren, die ferner aufgrund ihrer chemischen Natur Lichtschutzeigenschaften gegenüber UV-Strahlung aufweisen, und den Gemischen dieser Verbindungen.

10. Zusammensetzung nach einem der Ansprüche 1 bis 9, dadurch gekennzeichnet, daß das wasserlösliche UV-Filtersystem in Konzentrationen vorliegt, die vorzugsweise im Bereich von 0,1 bis 30 Gew.-% und insbesondere im Bereich von 0,5 bis 25 Gew.-%, bezogen auf das Gesamtgewicht der Zusammensetzung, liegen.

11. Zusammensetzung nach einem der Ansprüche 1 bis 10, dadurch gekennzeichnet, daß die Fettphase ein Öl oder mehrere Öle enthält, die ausgewählt sind unter:

    -   Mineralölen,
    -   Kohlenwasserstoffölen, die aromatische Ringe aufweisen,
    -   mehrfach veretherten Estern,
    -   Ölen tierischen Ursprungs,
    -   Ölen pflanzlichen Ursprungs,
    -   synthetischen Ölen,
    -   fluorierten und perfluorierten Ölen,
    -   Fettsäureestern,
    -   Fettalkoholen und
    -   gegebenenfalls organomodifizierten, geradkettigen, verzweigten oder cyclischen, flüchtigen oder nicht flüchtigen Siliconen.

12. Zusammensetzung nach einem der Ansprüche 1 bis 11, dadurch gekennzeichnet, daß sie als Pflegeprodukt, als Schminkprodukt oder als Produkt zu Haarbehandlung, das ausgespült wird oder im Haar verbleibt, verwendet wird.

13. Zusammensetzung nach einem der Ansprüche 1 bis 12, dadurch gekennzeichnet, daß sie als Produkt zum Schutz von Keratinsubstanzen und insbesondere der Haut gegen UV-Strahlung verwendet wird.

14. Kosmetisches Verfahren zum Schutz von menschlichen Keratinsubstanzen und insbesondere der Haut gegen UV-Strahlung, das darin besteht, eine wirksame Mengen einer in einem der Ansprüche 1 bis 13 definierten Zusammensetzung auf den Keratinträger aufzutragen.